# EUROPÄISCHE PATENTSCHRIFT

(11) **EP 1 817 338 B1**
(45) Veröffentlichungstag und Bekanntmachung des Hinweises auf die Patenterteilung: **30.03.2011**
(21) Anmeldenummer: 05784035.7
(22) Anmeldetag: 09.09.2005
(51) Int. Cl.: C07K 14/745

(54) **HERSTELLUNG EINES VON WILLENBRAND FAKTOR-PRÄPARATES MIT HOHER SPEZIFISCHER AKTIVITÄT**
PRODUCTION OF A VON WILLENBRAND FACTOR PREPARATION HAVING A GREAT SPECIFIC ACTIVITY
PRODUCTION D'UNE PREPARATION DE FACTEUR WILLEBRAND PRESENTANT UNE HAUTE ACTIVITE SPECIFIQUE

(30) Priorität: 14.09.2004 DE 102004044421
(43) Veröffentlichungstag der Anmeldung: 15.08.2007
(73) Patentinhaber: Biotest AG, D-63303 Dreieich (DE)
(72) Erfinder: KRETSCHMAR, Michael, 63500 Seligenstadt (DE); MÖLLER, Wolfgang, 61440 Oberursel (DE)
(74) Vertreter: Kalhammer, Georg
(86) Internationale Anmeldenummer: PCT/EP2005/009729
(87) Internationale Veröffentlichungsnummer: WO 2006/029774

(56) Entgegenhaltungen:
- US-A- 5 128 245
- FEDERICI AUGUSTO B: "The factor VIII/von Willebrand factor complex: basic and clinical issues." HAEMATOLOGICA. JUN 2003, Bd. 88, Nr. 6, Juni 2003 (2003-06), Seite EREP02, XP002356462 ISSN: 1592-8721
- GORMAN J J ET AL: "STUDIES ON THE STRUCTURE AND SUBUNIT COMPOSITION OF HUMAN ANTI HEMOPHILIC FACTOR" THROMBOSIS RESEARCH, Bd. 12, Nr. 2, 1978, Seiten 341-352, XP002356463 ISSN: 0049-3848
- SAUNDRY R H ET AL: "CHROMATOGRAPHY OF VWF ON DEXTRAN SULFATE SEPHAROSE" THROMBOSIS RESEARCH, Bd. 48, Nr. 6, 1987, Seiten 641-652, XP002356464 ISSN: 0049-3848
- JANSON JC, RYDEN L (EDTS): "Protein purification-principles, high-resolution methods and applications" 1998, WILEY-LISS , NEW YORK , XP002356473 Seite 190, Absatz 6 - Seite 191, Absatz 6 Seite 199; Abbildung 4.14 Seite 200, Absatz 1 - Absatz 2
- BERNARDI G: "Chromatography of proteins on hydroxyapatite" METHODS IN ENZYMOLOGY, Bd. 27, 1973, Seiten 471-479, XP009057844
- BARINGTON K A ET AL: "A VERY-HIGH-PURITY VON WILLEBRAND FACTOR PREPARATION CONTAINING HIGH-MOLECULAR-WEIGHT MULTIMERS" VOX SANGUINIS, S. KARGER AG, BASEL, CH, Bd. 76, März 1999 (1999-03), Seiten 85-89, XP000971400 ISSN: 0042-9007
- LETHAGEN S ET AL: "A COMPARITIVE IN VITRO EVALUATION OF SIX VON WILLEBRAND FACTOR CONCENTRATES" HAEMOPHILIA, BLACKWELL SCIENCE, OXFORD, GB, Bd. 10, Nr. 3, Mai 2004 (2004-05), Seiten 243-249, XP001182495 ISSN: 1351-8216
- BURNOUF-RADOSEVICH M ET AL: "CHROMATOGRAPHIC PREPARATION OF A THERAPEUTIC HIGHLY PURIFIED VON WILLEBRAND FACTOR CONCENTRATE FROM HUMAN CRYOPRECIPITATE" VOX SANGUINIS, S. KARGER AG, BASEL, CH, Bd. 62, Nr. 1, 1992, Seiten 1-11, XP009045809 ISSN: 0042-9007
- VEYRADIER A ET AL: "Laboratory diagnosis of von Willebrand disease" INTERNATIONAL JOURNAL OF CLINICAL AND LABORATORY RESEARCH, Bd. 28, Nr. 4, Dezember 1998 (1998-12), Seiten 201-210, XP009058399 ISSN: 0940-5437

## Beschreibung

Die Erfindung betrifft ein Verfahren zur Herstellung eines von Willbrand Faktor-Präparates mit gesteigerter spezifischer VWF-Aktivität, wobei Hydroxylapatit als Chromatographie-Medium verwendet wird.

Der von Willebrand Faktor ist ein Glykoprotein, das in Endothelzellen und Megakaryozyten synthetisiert wird. Das Molekulargewicht des Monomers beträgt ca. 225 000 Da. Durch Ausbildung von Disulfidbrücken werden in der Zelle Dimere ausgebildet, die wiederum zu Oligomeren aus bis zu 40 dimeren Untereinheiten ebenfalls über Disulfidverknüpfungen assoziieren. Die Konzentration des in das Plasma abgegebenen von Willebrand Faktors (VWF) beträgt 5-10 mg/l.

In der primären Hämostase hat der VWF die Aufgabe, die Adhäsion von Thrombozyten an verletztes Subendothel zu vermitteln und unter Bedingungen hoher Scherkräfte, wie im arteriellen System, die Thrombozytenaggregation zu unterstützen. Als Funktion in der sekundären Hämostase bindet VWF Faktor VIII, einen wichtigen Cofaktor bei der Blutgerinnung, in einem nicht kovalenten Komplex. Faktor VIII wird dadurch stabilisiert und vor vorzeitigem Abbau geschützt.

Das von Willebrand Syndrom (VWS) ist eine Bluterkrankheit, die durch quantitative oder qualitative Veränderung des VWF hervorgerufen wird. Häufig werden für die Behandlung von schweren Formen des VWS plasmatische Faktor VIII-Präparate eingesetzt, die einen relativ hohen Anteil an VWF enthalten. Blutungen können damit zwar gestillt werden, gleichzeitig steigt aber, besonders bei häufiger oder längerer Anwendung, das Thromboserisiko. Dies ist auf eine Überdosierung von Faktor VIII zurückzuführen. Wesentlich günstiger im Sinne der Patientensicherheit ist die Therapie von VWS-Kranken mit hochreinen, Faktor VIII-armen / -freien VWF-Konzentraten.

Häufiges Problem bei der Herstellung eines VWF-Präparates ist der Verlust an spezifischer VWF-Aktivität. Gemäß der o.g. Definition beträgt bei einer VWF-Konzentration von 5-10 µg/ml im Blut und einer definierten Aktivität von durchschnittlich 1 E/ml die spezifische VWF-Aktivität 100 - 200 E pro mg VWF-Antigen. In der Regel sinkt die spezifische VWF-Aktivität während der ersten Aufarbeitungsschritte bei der Herstellung von VWF-Präparaten drastisch. Strukturell korreliert dieser Verlust an spezifischer Aktivität mit proteolytischen Abbaureaktionen, die zur Erniedrigung des Anteils an hochmolekularen VWF-Molekülen (Multimeren) und zur Erhöhung des Anteils an niedermolekularen VWF-Molekülen (Dimeren / Tetrameren) führen.

In der Literatur werden verschiedene Verfahren beschrieben, die auf eine Erhöhung der spezifischen VWF-Aktivität zielen, um dieser Problematik entgegen zu wirken:

WO 98/38219 beschreibt ein Verfahren zur Gewinnung von VWF, bei dem VWF bei einer niedrigen Salzkonzentration an einen Kationenaustauscher gebunden wird und durch fraktionierte Elution VWF mit hoher spezifischer Aktivität gewonnen wird.

Die WO 96/10584 beschreibt ein Verfahren zur Gewinnung von hochreinem rekombinanten VWF mittels kombinierter Anionenaustausch-/Heparin-Affinitätschromatographie und die EP 0 705 846 die Trennung von hoch- und niedermolekularen Fraktionen von rekombinantem VWF mittels Heparin-Affinitätschromatographie.

Die Veröffentlichung A.B. Federici, Haematologica (Juni 2003), 88, Nr. 6, 3-12 beschreibt Zusammensetzungen, die VWF enthalten. US 5,128,254 offenbart die chromatographische Aufreinigung von Komponenten von Megakarayocyten oder Blutplättchen, wie den von Willebrand-Faktor.

Die Druckschrift Gorman und Ekert (1978), Thrombosis Research 12, 341-352 beschreibt Studien zur Struktur und zum Aufbau von humanem, antihämophilem Faktor. Dabei wurde der humane, antihämophile Faktor über Hydroxylapatit-Chromatographie, die einer Plasmapräzipitation und einer Gelfiltration folgte, aufgereinigt. Die Faktor VIII-Gerinnungsaktivität und die VWF-Aktivität eluierten gleichzeitig.

Die Veröffentlichung Saundry et al. (1987), Trombosis Research 48, 641-652 offenbart die chromatographische Aufreinigung von VWF:ag unter Verwendung von Dextran-Sulfat (DS) Agarose.

Barington und Kaersgaard (1999), Vox Sanguinis 76, 85-89 beschreibt Studien zur Bereitstellung von hochreinem von Willebrand-Faktor, der hochmolekulare Multimere enthält.

Burnouf-Radosevich und Burnouf (1992), Vox Sanguinis 62, 1-11 beschreibt Studien zur chromatographischen Aufbereitung eines gereinigten von Willebrand-Faktor-Konzentrats aus humanem Cryopräzipitat.

Die bisher beschriebenen Verfahren sind mit Nachteilen hinsichtlich Wirtschaftlichkeit und Reinigungseffektivität behaftet. Bei der Verwendung einer Heparin-Affinitätschromatographie zur Erhöhung der spezifischen VWF-Aktivität wurde beispielsweise die Aktivität einer vorgereinigten Proteinlösung, die rekombinanten VWF enthält, von 4,3 E pro mg VWF-Antigen auf 7,3 E/mg erhöht. Die finale spezifische VWF-Aktivität des rekombinanten Proteins ist niedrig. Die Kosten für eine Affinitätschromatographie sind vergleichsweise hoch. Bessere Ergebnisse wurden durch Anwendung einer Kationenaustauschchromatographie erzielt. Bei der Reinigung eines rekombinanten Antikörpers wurde in Kombination mit vorgeschalteter Anionenaustausch- und Immunaffinitätschromatographie eine spezifische VWF-Aktivität von 30,4 E/mg erzielt. Am Beispiel der Reinigung eines plasmatischen VWF durch die Kombination Anionenaustausch- und Kationenaustauschchromatographie wurde eine erzielte Reinheit von 65 E pro mg Protein erreicht. Auf die Steigerung der spezifischen VWF-Aktivität (Aktivität pro VWF-Antigenmenge) wurde nicht eingegangen.

Es besteht ein Bedürfnis nach einem wirtschaftlichen Verfahren, mit dem wenig aktive VWF-Moleküle aus einer Proteinfraktion abgereichert werden können, um so die spezifische Aktivität zu erhöhen. Dabei soll eine spezifische VWF-Aktivität erreicht werden, die der im Plasma gesunder Menschen entspricht. Erfindungsgemäß wird diese Aufgabe durch einen chromatographischen Separationsschritt unter Verwendung von Hydroxylapatit gelöst.

Die vorliegende Erfindung betrifft ein Verfahren zur Trennung von VWF mit hoher Aktivität von VWF mit niedriger Aktivität, umfassend eine Chromatographie mit Hydroxylapatit als Chromatographie-Matrix, wodurch eine VWF-Präparation mit hoher Aktivität erhalten wird, die eine spezifische VWF-Aktivität von wenigstens 50 E/mg VWF-Antigen aufweist. Ein weiterer Aspekt der Erfindung ist ein Verfahren zur Herstellung einer Zusammensetzung mit einer spezifischen VWF-Aktivität von wenigstens 50 E/mg VWF-Antigen, dadurch gekennzeichnet, dass man eine VWF enthaltende Zusammensetzung durch Hydroxylapatit-Chromatographie aufreinigt, wobei VWF an die Hydroxylapatit-Säulenmatrix gebunden wird, VWF mit niedriger spezifischer Aktivität herausgewaschen wird und anschließend VWF mit hoher spezifischer Aktivität bei höherer Salzkonzentration eluiert wird, wobei der Waschpuffer 100-300 mM, vorzugsweise 200-300 mM, und der Elutionspuffer 200-500 mM, vorzugsweise 300-400 mM Natrium- oder Kaliumphosphat enthält.

Hydroxylapatit ist eine Form von Calciumphosphat mit der Zusammensetzung Ca₅(PO₄)₃OH bzw. Ca₁₀(PO₄)₆OH₂, die als stationäre Phase für die Chromatographie von Proteinen, Nukleinsäuren und anderen Makromolekülen eingesetzt werden kann. Neben der kristallinen Form von Hydroxylapatit kann auch eine keramische Form verwendet werden, welche durch Sintern erhältlich ist. Hydroxylapatit kann beispielsweise von der Firma Bio-Rad (München, Deutschland) bezogen werden. Deren keramisches Hydroxylapatit wird in zwei Formen (Typ 1 und Typ 2) zur Verfügung gestellt. Typ 1-Material hat aufgrund größerer Oberflächen eine höhere Bindungskapazität für kleinere Moleküle, z.B. kleine Proteine. Dagegen weist das Typ 2-Material größere Poren in den Partikeln auf, die ein Eindringen und damit eine bessere Bindung von großen Molekülen, z.B. DNA oder großen Proteinen, ermöglicht. Die Materialien weisen vorzugsweise folgende Eigenschaften auf:

**Tabelle 1**

| | Dynamische Bindungskapazität | Nominale Porendurchmesser |
|---|---|---|
| Typ 1 | >13,7 mg Lysozym/ml CHT* | 600-800 Å |
| Typ 2 | >6,8 mg Lysozym/ml CHT* | 800-1000 Å |

| | | |
|---|---|---|
| *CHT=Ceramic Hydroxy Apatite | | |

Kristallines oder keramisches Hydroxylapatit ist frei verfügbar. Verfahren zu ihrer Herstellung sind im Stand der Technik bekannt. Im Rahmen der vorliegenden Erfindung ist die Verwendung von keramischem Hydroxylapatit bevorzugt.

Hydroxylapatit, insbesondere in seiner keramikstabilisierten Form ist außerordentlich gut geeignet, um Prozesse im Industriemaßstab durchzuführen. Aufgrund seiner Trenneigenschaften bietet Hydroxylapatit eine bessere Auflösung als die vielfach beschriebenen Ionenaustauschchromatographiemedien. Bei dem Hydroxylapatit-Reinigungsverfahren müssen den Puffern weder Calcium noch Aminosäuren zugesetzt werden.

Das Verfahren kann in Form einer Säulenchromatographie oder im Batch-Verfahren durchgeführt werden; die Durchführung einer Säulenchromatographie ist bevorzugt.

Üblicherweise umfasst das Verfahren, dass
(a) VWF an die Hydroxylapatit-Matrix gebunden wird,
(b) VWF mit niedriger spezifischer VWF-Aktivität bei mittlerer Salzkonzentration herausgewaschen wird; und
(c) anschließend VWF mit hoher spezifischer VWF-Aktivität bei höherer Salzkonzentration eluiert wird.

In Schritt (a) wird eine Lösung, die VWF hoher und niedriger spezifischer VWF-Aktivität enthält, mit der Hydroxylapatit-Matrix in Kontakt gebracht. Die Gesamtkonzentration an Natrium- und/oder Kaliumphosphat in dieser Lösung ist üblicherweise 0 bis 200 mM, vorzugsweise 1 bis 100 mM, bevorzugter 1 bis 50 mM, am bevorzugtesten 10 bis 30 mM.

In dem Waschschritt (b) wird die Hydroxylapatit-Matrix mit einem Puffer mittlerer Salzkonzentration gewaschen. Die Gesamtkonzentration an Natrium- und/oder Kaliumphosphat in diesem Waschpuffer ist in der Regel 100 bis 300 mM, vorzugsweise 200 bis 300 mM, bevorzugter 200 bis 270 mM, am bevorzugtesten 210 bis 250 mM. Dabei wird VWF mit niedriger spezifischer VWF-Aktivität herausgewaschen. Je höher der pH-Wert des Puffers, desto niedriger kann die Salzkonzentration gewählt werden.

In Schritt (c) kann VWF hoher spezifischer VWF-Aktivität mit einem Puffer höherer Salzkonzentration eluiert werden. Der Elutionspuffer enthält in der Regel 200 bis 500 mM, vorzugsweise 250 bis 500 mM, bevorzugter 300 bis 400 mM Natrium- und/oder Kaliumphosphat.

Durch Verschiebung der Salzkonzentrationen können Ausbeute und Reinheit verändert werden. Je höher die Salzkonzentration im Waschpuffer ist, desto aktiver ist die erhaltene Wertfraktion. Die Ausbeute erniedrigt sich dadurch allerdings. Weiterhin beeinflusst der gewählte pH-Wert die optimale Salzkonzentration für den Waschpuffer. Je niedriger der pH-Wert ist, desto stärker ist die Bindung von VWF an die Hydroxylapatit-Matrix. Entsprechend können die gewählten Salzkonzentrationen bei niedrigen pH-Werten höher sein, bei höheren pH-Werten hingegen niedriger.

Die Hydroxylapatit-Chromatographie wird bei einem pH-Wert von 5 bis 7, vorzugsweise von 5,5 bis 6,8, bevorzugter von 6,0 bis 6,8, am bevorzugtesten von 6,2 bis 6,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedliche pH-Werte aufweisen.

Je nach pH-Wert-Bedingungen und Ausbeute-Bestrebungen können wenig aktive VWF-Moleküle von Hydroxylapatit-Säulen mit Puffern eluiert werden, die eine mittlere Salzkonzentration enthalten. Bei pH 6,0 können beispielsweise Puffer verwendet werden, die 200 bis 270 mM Natrium- oder Kaliumphosphat, bevorzugt 220 bis 250 mM Natriumphosphat enthalten. Bei pH 6,5 wären beispielsweise Puffer geeignet, die 180 bis 260 mM, bevorzugt 210 bis 250 mM Natrium- oder Kaliumphosphat enthalten. Nach der Abtrennung der wenig aktiven VWF-Moleküle können die hochaktiven VWF-Moleküle mit einem Hochsalzpuffer, beispielsweise 250 bis 500 mM Natrium- oder Kaliumphosphat, bevorzugt 300 bis 400 mM Natrium- oder Kaliumphosphat eluiert werden.

Die erfindungsgemäße bindende Hydroxylapatit-Chromatographie kann mit anderen Reinigungstechniken kombiniert werden. Als besonders vorteilhaft hat es sich erwiesen, zunächst eine "Durchlaufchromatographie" mit Hydroxylapatit zur Abreicherung der Hauptverunreinigungen durchzuführen. Anschließend wird die Durchlauffraktion auf eine Hydroxylapatit-Säule aufgetragen, wie oben beschrieben. VWF-Moleküle werden gebunden und VWF hoher spezifischer VWF-Aktivität wird selektiv eluiert.

In einer besonderen Ausführungsform wird daher zunächst eine Durchlaufchromatographie mit Hydroxylapatit durchgeführt, wobei VWF nicht an die Hydroxylapatit-Matrix bindet, und anschließend die Durchlauffraktion unter bindenden Bedingungen rechromatographiert und die VWF-Fraktion mit VWF hoher spezifischer VWF-Aktivität eluiert.

Eine "Durchlaufchromatographie" im Sinne dieser Anmeldung umfasst, dass (i) eine Zusammensetzung, die VWF und ein oder mehrere verunreinigende Proteine enthält, mit einer Hydroxylapatit-Matrix in Kontakt gebracht wird, so dass wenigstens ein verunreinigendes Protein an die Hydroxylapatit-Matrix gebunden wird, während VWF im wesentlichen nicht an die Hydroxylapatit-Matrix gebunden wird, und gegebenenfalls anschließend (ii) ungebundenes VWF von der Hydroxylapatit-Matrix getrennt wird. Im Fall einer Säulenchromatographie befindet sich VWF im Durchlauf und wenigstens ein verunreinigendes Protein, z. B. Fibronektin und/oder Fibrinogen, wird an das Hydroxylapatit gebunden.

Die Durchlaufchromatographie wird bei einem pH-Wert von 6,5 bis 8,5, vorzugsweise von 6,8 bis 8,5, bevorzugter von 6,8 bis 7,5, am bevorzugtesten von 7,0 bis 7,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedlichen pH-Werte aufweisen.

Die VWF enthaltende Zusammensetzung, die bei der Durchlaufchromatographie mit der Hydroxylapatitmatrix in Kontakt gebracht wird, enthält vorzugsweise Natriumphosphat und/oder Kaliumphosphat. Die Gesamtkonzentration an Natriumphosphat und/oder Kaliumphosphat in der Lösung ist beispielsweise 0 bis 100 mM, vorzugsweise 10 bis 50 mM, am bevorzugtesten 20 bis 40 mM, d. h. als Laufpuffer kann eine Pufferlösung mit den genannten Konzentrationen eingesetzt werden.

Durch den vorgeschalteten Schritt der Durchlaufchromatographie können VWF-Präparationen erhalten werden, die nur geringe Mengen an Fibrinogen und Fibronektin enthalten. In der Regel ist die Konzentration an Fibrinogen-Antigen in der Durchlauffraktion niedriger als 25 µg/ml, bevorzugt niedriger als 15 µg/ml, bevorzugter niedriger als 10 µg/ml, am bevorzugtesten höchstens 5 µg/ml. Die Konzentration an Fibronektin-Antigen in der Durchlauffraktion ist üblicherweise niedriger als 250 µg/ml, bevorzugt niedriger als 150 µg/ml, bevorzugter niedriger als 100 µg/ml, am bevorzugtesten höchstens 50 µg/ml. Die Konzentration an Fibrinogen-Antigen und Fibronektin-Antigen kann durch an sich bekannte Verfahren bestimmt werden.

Durch den vorgeschalteten Schritt der Durchlaufchromatographie kann eine erhebliche Abreicherung der verunreinigenden Proteine Fibrinogen und Fibronektin erzielt werden. So ist die Konzentration an Fibrinogen in der Durchlauffraktion vorzugsweise niedriger als 10%, bevorzugter niedriger als 5%, noch bevorzugter niedriger als 2,5% der Konzentration an Fibrinogen in der Auftragslösung (vor Durchlaufchromatographie). Die Konzentration an Fibronektin in der Durchlauffraktion ist vorzugsweise niedriger als 10%, bevorzugter niedriger als 5%, noch bevorzugter niedriger als 2,5% der Konzentration an Fibronektin in der Auftragslösung (vor Durchlaufchromatographie).

Die Ausbeute der Durchlaufchromatographie (bezogen auf die Massenbilanz) ist in der Regel höher als 50%, bevorzugt höher als 60%, am bevorzugtesten höher als 75%. Für die Durchlaufchromatographie ist es sinnvoll, aber nicht notwendig, Phosphationen als Puffersubstanz zu verwenden. Für die Elution von VWF bei der bindenden Chromatographie ist Phosphat ein spezifisches Agens.

Als Ausgangsmaterialien können allgemein VWF-haltige Lösungen, im Besonderen VWF-haltige Plasmafraktionen eingesetzt werden. VWF kann bei pH-Werten von vorzugsweise 6,0 bis 6,8, besonders bevorzugt 6,2 bis 6,5, bei einer niedrigen Salzkonzentration von 0-100 mM Kalium- oder Natriumphosphat, bevorzugt bei 10-50 mM an eine Hydroxylapatit-Säule gebunden werden.

In einer besonderen Ausführungsform kann anstelle von reinem Hydroxylapatit als Chromatographiematrix Fluoroapatit verwendet werden. Fluoroapatit wird durch Umsetzung von Hydroxylapatit mit einer fluoridhaltigen Substanz hergestellt. Die Firma Bio-Rad (München, Deutschland) erzeugt z.B. keramisches Fluoroapatit durch eine 90%-ige Umsetzung von keramischem Hydroxylapatit mit einem Fluorinreagenz. Fluoropatit ist im Vergleich zu Hydroxylapatit unter sauren pH-Wertbedingungen deutlich stabiler. Deshalb wird Fluoroapatit üblicherweise eingesetzt, um Chromatographien bei niedrigerem pH durchzuführen, als für Hydroxylapatit technisch sinnvoll. Für eine Aufreinigung von hochaktiven VWF-Molekülen mit Fluoroapatit kann die Chromatographie ähnlich zu dem für Hydroxylapatit vorgeschlagenen Verfahren durchgeführt werden. Salzkonzentrationen müssen dem gewählten pH-Wert (>= 5,0) angepasst werden.

Das erfindungsgemäße Verfahren kann weiterhin einen oder mehrere der folgenden Schritte umfassen:
(1) Schockgefrieren bei einer Temperatur von unter -30°C und Auftauen nahe 0°C (Kryopräzipitation)
(2) Ethanolfällung oder Adsorption an Aluminiumhydroxid
(3) Virusinaktivierung der VWF enthaltenden Zusammensetzung durch Solvens/Detergens-Behandlung
(4) Anionenaustauchchromatographie
(5) Fällung von Fibronektin durch Einstellung eines pH-Werts von unter pH 5,4
(6) Affinitätschromatographie
(7) Diafiltration oder Ultrafiltration
(8) Umpuffern durch Dialyse oder Gelfiltration
(9) Sterilfiltration
(10) Lyophilisierung
(11) Virusinaktivierung durch Hitzebehandlung (z.B. ca. 30 min bei ca. 100°C)

Die Verfahrensschritte (1) bis (5) werden vorzugsweise vor der Hydroxylapatit-Chromatographie durchgeführt. Es können aber auch weniger als die 5 Verfahrensschritte durchgeführt werden. Die Reihenfolge der Schritte ist nicht zwingend.

Die Schritte (6) bis (11) können durchgeführt werden, falls gewünscht. Insbesondere die Affinitätschromatographie ist aber nicht notwendig, da bereits durch die Hydroxylapatitchromatographie eine hohe Reinheit erreicht werden kann.

Als Ausgangsmaterial für die Verfahren der vorliegenden Erfindung kann somit eine vorgereinigte Plasmafraktion verwendet werden. Hierbei kann es sich um eine weiter aufgereinigte Kryopräzipitat-Lösung handeln. Die Kryopräzipitat-Lösung kann beispielsweise mit Aluminiumhydroxid gefällt werden und/oder chromatographisch weiter aufgereinigt werden. So kann die Kryopräzipitat-Lösung z. B. mit Aluminiumhydroxid gefällt werden und anschließend mittels Anionenaustauschchromatographie weiter aufgereinigt werden. Es ist ebenfalls bevorzugt, dass die Kryopräzipitat-Lösung einer Virusinaktivierung unterworfen wird. Bevorzugtes Verfahren zur Virusinaktivierung ist eine Solvens/DetergensBehandlung, wie sie im US-Patent Nr. 4,540,573 beschrieben ist.

Als Ausgangsmaterial kann ebenso eine Proteinlösung enthaltend rekombinanten VWF (rVWF) aus Zellkulturüberständen verwendet werden. Der Ausdruck "rVWF" umfasst sowohl VWF mit der Wildtypsequenz als auch Varianten mit einer gegenüber Wildtyp-VWF veränderten Aminosäuresequenz, wobei ein oder mehrere Aminosäuren subsituiert, deletiert und/oder hinzugefügt sein können. Die Varianten weisen in der Regel VWF-Aktivität auf. Verfahren zur Herstellung geeigneter Expressionsvektoren, zur Einschleusung der Vektoren in die Wirtszellen und zur Kultivierung der Wirtszellen sind dem Fachmann an sich bekannt (Fischer et al. Structural analysis of recombinant von Willebrand factor: identification of hetero and homo-dimers. FEBS Lett 1994; 351:345-348. Fischer et al. Structural analysis of recombinant von Willebrand factor produced at industrial scale fermentation of transformed CHO cells co-expressing recombinant furin. FEBS Lett 1995; 375:259-262).

Insbesondere bei Verwendung von Plasmafraktionen kann vor der Hydroxylapatit-Chromatographie eine pH-Fällung zur Abtrennung von Fibronektin durchgeführt werden. Die pH-Fällung zur Abtrennung von Fibronektin aus einer Plasmafraktion umfasst beispielsweise, dass man
(i) den pH-Wert der Plasmafraktion auf unter pH 5,4 einstellt, so dass sich ein Niederschlag bildet und
(ii) den gebildeten Niederschlag abtrennt.

Der Ausdruck "Plasmafraktion" bezeichnet in diesem Zusammenhang eine Zusammensetzung, die aus Plasma erhalten wurde und verschiedene Plasmaproteine enthält. Die Plasmafraktion, die als Ausgangszusammensetzung in Schritt (i) eingesetzt wird, ist eine flüssige Zusammensetzung. Vorzugsweise ist die flüssige Zusammensetzung eine Lösung oder eine Suspension, am bevorzugtesten ist die Zusammensetzung eine Lösung. In einer besonderen Ausführungsform ist die Plasmafraktion gelöstes Kryopräzipitat. Dieses gelöste Kryopräzipitat kann durch verschiedene Verfahren vorgereinigt sein. Beispiele sind Aluminiumhydroxid-Behandlung, Solvens-/DetergensBehandlung und/oder Anionenaustauschchromatographie. Die Konzentration an Natriumchlorid oder Kaliumchlorid in der Plasmafraktion ist vorzugsweise 50 bis 250 mM, bevorzugter 100 bis 200 mM, am bevorzugtesten 120 bis 150 mM. Die Plasmafraktion kann beispielsweise folgende Puffersubstanzen enthalten: Citrationen, Acetationen, Phosphationen und/oder Aminosäuren.

Die Konzentration an Fibronektin in der Plasmafraktion, die Schritt (i) unterworfen wird, ist in der Regel mindestens 0,05 g/l, vorzugsweise wenigstens 0,1 g/l, noch bevorzugter wenigstens 0,25 g/l, am bevorzugtesten wenigstens 0,5 g/l. Die Konzentration an Fibronektin in der Plasmafraktion kann beispielsweise 0,1 bis 5 g/l, vorzugsweise 0,1 bis 2 g/l sein.

Zur Abtrennung von Fibronektin aus der Plasmafraktion wird der pH-Wert der Plasmafraktion auf unter pH 5,4 eingestellt. Dabei bildet sich ein Niederschlag, der Fibronektin enthält. Vorzugsweise wird der pH-Wert auf unter pH 5,3 eingestellt, noch bevorzugter auf unter pH 5,2. Der eingestellte pH-Wert liegt somit vorzugsweise in einem Bereich von pH 4,5 bis unter 5,4, bevorzugt in einem Bereich von pH 4,7 bis 5,3, bevorzugter in einem Bereich von pH 4,8 bis 5,2, noch bevorzugter in einem Bereich von pH 4,9 bis 5,1. In der Regel wird das Einstellen des pH-Werts durch Zugabe einer sauren Komponente erreicht. Als saure Komponente können verschiedene Säuren eingesetzt werden, beispielsweise Salzsäure, Phosphorsäure oder Essigsäure. Die saure Komponente wird üblicherweise über einen bestimmten Zeitraum zugegeben, beispielsweise tropfenweise. Somit wird nach und nach ein pH-Wert im oben näher definierten Bereich eingestellt ("titriert").

Während und nach dem Einstellen des pH-Werts wird die Plasmafraktion vorzugsweise in Bewegung gehalten oder durchmischt, beispielsweise durch Rühren. Es ist weiterhin bevorzugt, dass nach Einstellen des pH-Werts die Plasmafraktion für einen bestimmten Zeitraum weiter durchmischt wird (z.B. durch Rühren), im allgemeinen für wenigstens 10 Minuten, vorzugsweise für wenigstens 20 Minuten, am bevorzugtesten für einen Zeitraum von 30 bis 90 Minuten. In diesem Zeitraum bilden sich klebrige Aggregate, die zu einem erheblichen Anteil Fibronektin erhalten. Daher ist es gemäß einer bevorzugten Ausführungsform vorgesehen, dass ein geeigneter Rührer, z.B. ein Anker- oder Paddelrührer eingesetzt wird, an dessen Rührblatt sich der Niederschlag anheftet. Das präzipitierte Fibronektin kann somit leicht der Lösung entzogen werden.

Die pH-Fällung zur Abtrennung von Fibronektin kann in einem breiten Temperaturspektrum durchgeführt werden, z.B. von ca. 1 °C bis ca. 37°C. Bevorzugte Temperaturbereiche sind 4 bis 35°C, bevorzugter 10 bis 30°C, am bevorzugtesten wird das Verfahren bei 20 bis 25°C durchgeführt.

Durch die pH-Fällung zur Abtrennung von Fibronektin aus Plasmafraktionen kann die Konzentration an Fibronektin in der Plasmafraktion um wenigstens 50% verringert werden. Vorzugsweise wird die Konzentration an Fibronektin in der Plasmafraktion um 70 bis 99%, bevorzugter um 80 bis 99%, am bevorzugtesten um 90 bis 98% oder um 95 bis 98% verringert. In einer besonderen Ausführungsform liegt der Verlust an VWF in dem Fällungsschritt bei höchstens 50%, bevorzugt bei höchstens 40%, bevorzugter bei höchstens 30%, noch bevorzugter bei höchstens 20%, am bevorzugtesten bei höchstens 10%.

Beschrieben wird weiterhin eine VWF enthaltende Zusammensetzung erhältlich durch ein erfindungsgemäßes Verfahren, wie es in der vorliegenden Anmeldung beschrieben ist. Vorzugsweise handelt es sich um eine im wesentlichen reine VWF-Präparation. "Im wesentlichen rein" bedeutet, dass die Zusammensetzung im wesentlichen frei von anderen Proteinen ist, insbesondere dass kein Fibronektin und kein Fibrinogen nachweisbar ist.

Die VWF-Präparation kann eine spezifische Aktivität von wenigstens 75 E/mg Protein aufweisen, vorzugsweise hat sie eine spezifische Aktivität von wenigstens 85 E/mg Protein, bevorzugter von wenigstens 100 E/mg Protein, am bevorzugtesten von wenigstens 120 E/mg Protein. Durch das erfindungsgemäße Verfahren können somit spezifische Aktivitäten von >100 E pro mg Protein erzielt werden. Die VWF-Aktivität kann bestimmt werden wie in den Beispielen beschrieben. Durch die erfindungsgemäße Hydroxylapatit-Chromatographie kann die spezifische Aktivität (E/mg Protein) um wenigstens 100%, bevorzugt um wenigstens 150%, am bevorzugtesten um wenigstens 175% erhöht werden.

Die erfindungsgemäße Zusammensetzung hat vorzugsweise eine spezifische VWF-Aktivität von wenigstens 50 E/mg VWF-Antigen, bevorzugter von wenigstens 75 E/mg VWF-Antigen, noch bevorzugter von wenigstens 100 E/mg VWF-Antigen, noch bevorzugter von wenigstens 120 E/mg VWF-Antigen, noch bevorzugter von wenigstens 125 E/mg VWF-Antigen, am bevorzugtesten von wenigstens 150 E/mg VWF-Antigen. Durch die erfindungsgemäße Hydroxylapatit-Chromatographie kann die spezifische VWF-Aktivität (E/mg VWF-Antigen) um wenigstens 20%, bevorzugt um wenigstens 35%, am bevorzugtesten um wenigstens 50% erhöht werden.

Ein weiterer Aspekt der Erfindung ist die Verwendung von Hydroxylapatit zur Herstellung eines VWF-Präparats mit hoher spezifischer VWF-Aktivität. Die Erfindung betrifft weiterhin die Verwendung von Hydroxylapatit zur Trennung von VWF hoher spezifischer VWF-Aktivität von VWF niedriger spezifischer VWF-Aktivität. Die Anmeldung beschreibt weiterhin die Verwendung von Hydroxylapatit zur Erhöhung der spezifischen VWF-Aktivität einer VWF enthaltenden Zusammensetzung. Die bevorzugten Ausführungsformen der erfindungsgemäßen Verwendung entsprechen denen des erfindungsgemäßen Verfahrens und der erfindungsgemäßen Zusammensetzung.

Die verschiedenen in der vorliegenden Anmeldung beschriebenen Ausführungsformen können miteinander kombiniert werden.

Die nachfolgenden Beispiele erläutern die Erfindung näher.

### Beispiel 1: Erhöhung der spezifischen VWF-Aktivität einer vorgereinigten VWF-haltigen Plasmafraktion mittels Hydroxylapatit-Chromatographie im Labormaßstab

Die VWF-haltige Proteinlösung durchlief folgende Vorreinigungsstufen: Eine Kryopräzipitat-Lösung wurde einer Aluminiumhydroxid-Fällung unterzogen. Anschließend wurde eine Virusinaktivierung mittels S/D-Behandlung durchgeführt. Bei der anschließenden Anionenaustauschchromatographie fällt eine VWF-haltige Waschfraktion an, die vor allem mit Fibrinogen und Fibronektin verunreinigt ist. Die Proteinlösung wird durch Titration auf pH 5.2 einem Fällungsschritt unterzogen, bei dem ein großer Teil des Fibronektins und Fibrinogens entfernt wurde. Anschließend wurde eine Ultra- und Diafiltration durchgeführt, wobei die Proteinlösung ca. 7-fach ankonzentriert und gegen den Laufpuffer der folgenden Chromatographie diafiltriert wurde. Die so erhaltene Proteinlösung enthielt ca. 930 µg/ml VWF-Antigen, 270 µg/ml Fibrinogen-Antigen und 2400 µg/ml Fibronektin-Antigen. Die Proteinlösung wurde auf eine in 10 mM Na-Phosphat pH 7,0 equilibrierte Hydroxylapatit-Säule (CHT Typ 1, Bio-Rad, München, Deutschland) aufgetragen. Die Durchlauffraktion enthielt ca. 560 µg/ml VWF-Antigen, 5 µg/ml Fibrinogen-Antigen und 50 µg/ml Fibronektin-Antigen. Die Massenbilanz ergibt eine Schrittausbeute von 78% für VWF-Antigen und 73% VWF-Aktivität.

Eine so vorgereinigte Proteinlösung wurde zur weiteren erfindungsgemäßen Aufreinigung auf pH 6,0 titriert. Anschließend wurde die Lösung auf eine Hydroxylapatit-Säule (CHT Typ 1, Bio-Rad, München, Deutschland) aufgetragen, die im Laufpuffer (20 mM Na-Phosphat pH 6,0) equilibriert worden war. Der VWF wurde quantitativ gebunden. Eine erste Elution wurde mit 45% des Elutionspuffers B (400 mM Na-Phosphat pH 6,0) durchgeführt, die zweite mit 100% B. Die aufgetragene Proteinlösung enthielt 540 µg/ml VWF-Antigen, 4 µg/ml Fibrinogen und 48 µg/ml Fibronektin. Die Aktivität des VWF in dieser Lösung betrug 46 E/ml. Die erste Elutionsfraktion enthielt 38 µg/ml VWF-Antigen, 2 µg/ml Fibronektin-Antigen und hatte eine VWF-Aktivität von 0,7 E/ml. Die Fibrinogen-Antigen-Konzentration lag unterhalb des Detektionslimits von 1 µg/ml. Die Wertfraktion (2. Elutionsfraktion) wies eine VWF-Antigen-Konzentration von 173 µg/ml, eine VWF-Aktivität von 23 E/ml auf. Weder Fibrinogen-Antigen, noch Fibronektin-Antigen konnte detektiert werde. Die Restaktivität von belegt, dass speziell wenig aktive VWF-Moleküle abgetrennt werden. Die Ausbeute an VWF-Aktivität liegt bei 84%, die an VWF-Antigen bei 51 %.

**Tabelle 2: Erhöhung der spezifischen VWF-Aktivität einer vorgereinigten VWF-haltigen Plasmafraktion mittels Hydroxylapatit-Chromatographie im Labormaßstab**

| | VWF-Antigen [µg/ml] | VWF-Aktivität [E/ml] | Spezifische VWF-Aktivität [E pro mg VWF-Antigen] |
|---|---|---|---|
| Ausgangsmaterial aus Chrom. I | 560 | 46 | 85 |
| Elution 1 Chrom. II | 38 | 0,7 | 18 |
| Elution 2 Chrom. II | 173 | 23 | 133 |

Die Konzentration an VWF-Antigen wurde mit Hilfe des STA^{®} Compact der Firma Diagnostica Stago (Roche Diagnostics, Mannheim) und deren Test-Reagenzien (STA LIA vWF) bestimmt.

Die VWF-Aktivität wurde als Ristocetin-Cofaktor-Aktivität über Plättchenagglutination mit dem BCT^{®}-Analyser (Behring Coagulation Timer, Dade Behring, Schwalbach) bestimmt. Der Ristocetin-Cofaktor-Assay ermittelt die Bindungsfähigkeit von VWF an den Plättchenrezeptor Glycoprotein Ib/IX unter dem Einfluss des Antibiotikums Ristocetin.

### Beispiel 2: Erhöhung der spezifischen VWF-Aktivität einer vorgereinigten VWF-haltigen Plasmafraktion mittels Hydroxylapatit-Chromatographie im Technikumsmaßstab

Die Ausgangslösung wurde entsprechend Beispiel 1 bis zur ersten Chromatographie präpariert. Die so erhaltene Proteinlösung enthielt ca. 1,43 mg/ml VWF-Antigen bei einer Proteingesamtkonzentration von 3,79 mg/ml. Die Spezifische Aktivität betrug 36,4 E / mg Protein. Für die chromatographische Reinigung wurden Säulen mit einem Durchmesser von 10 cm verwendet. Die Proteinlösung wurde in einem ersten chromatographischen Reinigungsschritt auf eine in 10 mM Na-Phosphat pH 7,0 equilibrierte Hydroxylapatit-Säule (CHT Typ 1, Bio-Rad, München, Deutschland) mit 1,0 I Gelbettvolumen aufgetragen. Die Durchlauffraktion (Wertfraktion 1) enthielt ca. 0,67 mg/ml VWF-Antigen bei einer Proteingesamtkonzentration von 0,86 mg/ml. Die Spezifische Aktivität betrug 68,7 E/mg Protein und die Spezifische VWF-Aktivität 96,5 E / mg VWF-Antigen.

Eine so vorgereinigte Proteinlösung wurde zur weiteren Aufreinigung und zur Abtrennung von wenig aktiven VWF-Molekülen auf pH 6,5 titriert. Anschließend wurde die Lösung auf eine Hydroxylapatit-Säule (CHT Typ 2, Bio-Rad, München, Deutschland) mit einem Gelbettvolumen von 1,5 I aufgetragen, die im Laufpuffer (20 mM Na-Phosphat pH 6,5) equilibriert worden war. Der VWF wurde quantitativ gebunden. Eine erste Elution zur Abtrennung von wenig aktiven VWF-Molekülen wurde mit 220 mM Na-Phosphat pH 6,5 durchgeführt. Die Wertfraktion 2 mit einer Proteinkonzentration von 0,20 mg/ml und einem Gehalt an VWF-Antigen von 0,13 mg/ml wurde im Anschluss mit 300 mM Na-Phosphat pH 6,5 eluiert. Durch diesen chromatographischen Schritt konnte die spezifische VWF-Aktivität von 87,5 E / mg VWF-Antigen auf 171 E/mg VWF-Antigen erhöht werden. Die spezifische Aktivität wurde dabei auf 107 E / mg Protein gesteigert. Verunreinigungen wie Fibronektin, Fibrinogen oder Faktor VIII lagen unterhalb des Detektionslimits.

Die Veränderung der Spezifischen VWF-Aktivitäten während des Reinigungsprozesses (siehe Tabelle 3) zeigt, dass während der ersten Chromatographie dieser Parameter nicht positiv beeinflusst wird. Der erfindungsgemäße zweite Chromatographische Schritt ist dagegen hervorragend geeignet, die Spezifische VWF-Aktivität zu steigern, was einer Abtrennung von wenig aktiven VWF-Molekülen entspricht.

**Tabelle 3: Erhöhung der spezifischen VWF-Aktivität einer vorgereinigten VWF-haltigen Plasmafraktion mittels Hydroxylapatit-Chromatographie im Technikumsmaßstab**

| | VWF-Antigen [mg/ml] | Spezifische Aktivität [E pro mg Protein] | Spezifische VWF-Aktivität [E pro mg VWF-Antigen] |
|---|---|---|---|
| Ausgang | 1,43 | 36,4 | 96,5 |
| Wertfraktion 1 | 0,67 | 68,7 | 87,5 |
| Wertfraktion 2 | 0,13 | 107 | 171 |

VWF-Antigen und VWF-Aktivität wurden bestimmt wie in Beispiel 1 angegeben.

Die Proteinbestimmung wurde nach dem Lambert-Beerschen Gesetz (A=ε·c·d) durchgeführt, wobei
A=Absorption bei 280 nm
ε=Absorptionskoeffizient (hier theoretischer Absorptionskoeffizient 0,75 cm²/mg)
c=Proteinkonzentration in mg/ml
d=Schichtdicke in cm.

### Beispiel 3: Erhöhung der spezifischen VWF-Aktivität einer vorgereinigten VWF-haltigen Plasmafraktion mittels Fluoroapatit-Chromatographie

Die Ausgangslösung wurde entsprechend Beispiel 1 bis zur zweiten Chromatographie präpariert. Die Proteinlösung enthielt bei einer Proteinkonzentration von 0,83 mg/ml eine spezifische Aktivität von 64,8 E/mg und eine spezifische VWF-Aktivität von 95,1 mg/ml. Durch Zugabe von HCl wurde die Proteinlösung auf einen pH-Wert von 6,0 titriert. Anschließend wurde die Lösung auf eine Fluoroapatit-Säule (CFT Typ 1, Bio-Rad, München, Deutschland) mit einem Gelbettvolumen von 13,2 ml aufgetragen, die im Laufpuffer (20 mM Na-Phosphat pH 6,0) equilibriert worden war. Ein Teil des VWF wurde gebunden. Eine Abtrennung von wenig aktiven VWF-Molekülen wurde durch eine erste Elution mit einem Puffer erzielt, der 241 mM Na-Phosphat pH 6,0 enthielt (Wertfraktion 1). Die Wertfraktion 2 mit einer Proteinkonzentration von 0,12 mg/ml und einem Gehalt an VWF-Antigen von 0,06 mg/ml wurde im Anschluss mit 400 mM Na-Phosphat pH 6,0 eluiert. Durch diesen chromatographischen Schritt konnte die spezifische VWF-Aktivität auf 152 E / mg VWF-Antigen erhöht werden. Die spezifische Aktivität wurde dabei auf 77 E / mg Protein gesteigert. Aufgrund der schwächeren Bindung von VWF bei den gewählten pH-Bedingungen findet keine komplette Bindung des VWF statt, so dass sich ein Teil in der Durchlauffraktion befindet.

**Tabelle 4: Erhöhung der spezifischen VWF-Aktivität einer vorgereinigten VWF-haltigen Plasmafraktion mittels Fluoroapatit-Chromatographie**

| | VWF-Antigen [mg/ml] | Spezifische Aktivität [E pro mg Protein] | Spezifische VWF-Aktivität [E pro mg VWF- Antigen] |
|---|---|---|---|
| Ausgang | 0,57 | 64,8 | 95,1 |
| Durchlauf | 0,07 | 47,1 | 89 |
| Wertfraktion 1 | 0,06 | 39,3 | 71,7 |
| Wertfraktion 2 | 0,06 | 77 | 152 |

### Ergänzende Literaturstellen

Folgende Literaturstellen zu verschiedenen Analytikmethoden werden ergänzend genannt:

### VWF-Aktivität:

Veyradier A, Fressinaud E, Meyer D (1998): Laboratory diagnosis of von Willebrand disease. Int J Lab Res 28 (4): 201-210.

### VWF-Antigen:

Budde U, et al. (1984): Acquired von Willebrand's disease in the myeloproliferative syndrome. Blood 64 (5): 981-985.
Newman DJ, Henneberry H, Price CP (1992): Particle enhanced light scattering immunoassay. Ann Clin Biochem 29 (Pt1): 22-42.

### Fibronektin-Antigen:

Sandberg L, et al. (1985): Plasma fibronectin levels in acute and recovering malnourished children. Clin Physiol Biochem. 3(5):257-264.
Colli A, et al. (1986): Diagnostic accuracy of fibronectin in the differential diagnosis of ascites. Cancer.: 58(11):2489-2493.

### Fibrinogen-Antigen:

Ernst E, Resch KL (1993): Fibrinogen as a cardiovascular risk factor: a meta-analysis and review of the literature. Ann Intern Med.: 118(12):956-963.
Jelic-Ivanovic Z, Pevcevic N (1990): Fibrinogen determination by five methods in patients receiving streptokinase therapy. Clin Chem.: 36(4):698-699.

## Patentansprüche

1. Verfahren zur Trennung von VWF mit hoher Aktivität von VWF mit niedriger Aktivität, umfassend eine Chromatographie mit Hydroxylapatit als Chromatographie-Matrix, wodurch eine VWF-Präparation mit hoher Aktivität erhalten wird, die eine spezifische VWF-Aktivität von wenigstens 50 E/mg VWF-Antigen aufweist.

2. Verfahren nach Anspruch 1, **dadurch gekennzeichnet, dass** der Waschpuffer 100-300 mM, vorzugsweise 200-300 mM, und der Elutionspuffer 200-500 mM, vorzugsweise 300-400 mM Natrium- oder Kaliumphosphat enthält.

3. Verfahren zur Herstellung einer Zusammensetzung mit einer spezifischen VWF-Aktivität von wenigstens 50 E/mg VWF-Antigen, **dadurch gekennzeichnet, dass** man eine VWF enthaltende Zusammensetzung durch Hydroxylapatit-Chromatographie aufreinigt, wobei VWF an die Hydroxylapatit-Säulenmatrix gebunden wird, VWF mit niedriger spezifischer Aktivität herausgewaschen wird und anschließend VWF mit hoher spezifischer Aktivität bei höherer Salzkonzentration eluiert wird, wobei der Waschpuffer 100-300 mM, vorzugsweise 200-300 mM, und der Elutionspuffer 200-500 mM, vorzugsweise 300-400 mM Natrium- oder Kaliumphosphat enthält.

4. Verfahren nach einem der Ansprüche 1 bis 3, **dadurch gekennzeichnet, dass** die Chromatographie bei einem pH-Wert zwischen 5 und 7, vorzugsweise zwischen 5,5 und 6,8 durchgeführt wird.

5. Verfahren nach einem der Ansprüche 1 bis 4, **dadurch gekennzeichnet, dass** als Laufpuffer eine Natrium- bzw. Kaliumphosphat-haltige Lösung verwendet wird.

6. Verfahren nach einem der Ansprüche 1 bis 5, **dadurch gekennzeichnet, dass** zunächst eine Durchlaufchromatographie mit Hydroxylapatit durchgeführt wird, die Durchlauffraktion unter bindenden Bedingungen rechromatographiert und das Zielprotein als hochreine VWF-Fraktion eluiert wird.

7. Verfahren nach einem der Ansprüche 1 bis 6, **dadurch gekennzeichnet, dass** man keramisches Hydroxylapatit verwendet.

8. Verfahren nach Anspruch 7, **dadurch gekennzeichnet, dass** das keramische Hydroxylapatit vom Typ I bzw. Typ II ist.

9. Verfahren nach einem der Ansprüche 1 bis 8, **dadurch gekennzeichnet, dass** als Ausgangsmaterial eine vorgereinigte Plasmafraktion verwendet wird.

10. Verfahren nach einem der Ansprüche 1 bis 9, **dadurch gekennzeichnet, dass** als Ausgangsmaterial eine weiter aufgereinigte Kryopräzipitat-Lösung verwendet wird.

11. Verfahren nach einem der Ansprüche 1 bis 10, **dadurch gekennzeichnet, dass** als Ausgangsmaterial eine mit Aluminiumhydroxid gefällte Kryopräzipitat-Lösung verwendet wird.

12. Verfahren nach einem der Ansprüche 1 bis 11, **dadurch gekennzeichnet, dass** als Ausgangsmaterial eine chromatographisch vorgereinigte Aluminiumhydroxid-gefällte Kryopräzipitat-Lösung verwendet wird.

13. Verfahren nach einem der Ansprüche 1 bis 12, **dadurch gekennzeichnet, dass** vor der Hydroxylapatit-Chromatographie eine pH-Fällung zur Abtrennung von Fibronektin durchgeführt wird.

14. Verfahren nach einem der Ansprüche 1 bis 8, **dadurch gekennzeichnet, dass** als Ausgangsmaterial eine Proteinlösung mit rekombinant hergestelltem VWF verwendet wird.

15. Verwendung von Hydroxylapatit zur Herstellung eines VWF-Präparats mit hoher spezifischer Aktivität, die wenigstens 50 E/mg VWF-Antigen beträgt.

## Claims

1. A process for separating VWF having a high activity from VWF having a low activity, comprising a chromatography with hydroxylapatite as a chromatography matrix, whereby a VWF preparation with high activity is obtained having a specific VWF activity of at least 50 U/mg VWF antigen.

2. The process according to claim 1, **characterized in that** the wash buffer contains 100 - 300 mM, preferably 200 - 300 mM, and the elution buffer contains 200 - 500 mM, preferably 300 - 400 mM, sodium or potassium phosphate.

3. A process for the production of a composition having a specific VWF activity of at least 50 U/mg VWF antigen, **characterized in that** a VWF containing composition is purified by means of hydroxylapatite chromatography wherein VWF is bound to the hydroxylapatite column matrix, VWF having a low specific activity is washed out and then VWF having a high specific activity is eluted at a relatively high salt concentration, and wherein the wash buffer contains 100 - 300 mM, preferably 200 - 300 mM, and the elution buffer contains 200 - 500 mM, preferably 300 - 400 mM, sodium or potassium phosphate.

4. The process according to any of claims 1 to 3, **characterized in that** the chromatography is carried out at a pH between 5 and 7, preferably between 5.5 and 6.8.

5. The process according to any of claims 1 to 4, **characterized in that** a sodium or potassium phosphate containing solution is used as a running buffer.

6. The process according to any of claims 1 to 5, **characterized by** initially carrying out flow chromatography with hydroxylapatite, rechromatographing the flow fraction under binding conditions and eluting the target protein as a highly pure VWF fraction.

7. The process according to any of claims 1 to 6, **characterized in that** ceramic hydroxylapatite is used.

8. The process according to claim 7, **characterized in that** the ceramic hydroxylapatite is type I or type II.

9. The process according to any of claims 1 to 8, **characterized in that** a previously purified plasma fraction is used as the starting material.

10. The process according to any of claims 1 to 9, **characterized in that** a further purified cryoprecipitate solution is used as the starting material.

11. The process according to any of claims 1 to 10, **characterized in that** a cryoprecipitate solution precipitated with aluminum hydroxide is used as the starting material.

12. The process according to any of claims 1 to 11, **characterized in that** a chromatographically pre-purified cryoprecipitate solution precipitated with aluminum hydroxide is used as the starting material.

13. The process according to any of claims 1 to 12, **characterized in that** a pH precipitation is carried out prior to the hydroxylapatite chromatography to separate fibronectin.

14. The process according to any of claims 1 to 8, **characterized in that** a protein solution with recombinantly produced VWF is used as the starting material.

15. Use of hydroxylapatite for the production of a VWF preparation having a high specific VWF activity of at least 50 U/mg VWF antigen.

## Revendications

1. Procédé de séparation du VWF à forte activité et du VWF à faible activité, comprenant une chromatographie sur hydroxylapatite comme matrice chromatographique, par laquelle une préparation de VWF à forte activité est conservée, qui indique une activité de VWF spécifique d'au moins 50 E/mg de VWF antigène.

2. Procédé conforme à la revendication 1, **caractérisé par le fait que** le tampon de lavage de 100-300 mM, de préférence de 200-300 mM, et le tampon d'élution de 200-500 mM, de préférence de 300-400 mM, contient du phosphate de sodium ou de potassium.

3. Procédé de production d'une composition de VWF ayant une activité spécifique d'au moins 50 E/mg de VWF antigène, **caractérisé par le fait que** l'on purifie une composition contenant du VWF par chromatographie sur hydroxylapatite, à travers laquelle le VWF est lié à la matrice colonne d'hydroxylapatite, le VWF à faible activité spécifique est lavé et, enfin, le VWF à forte activité spécifique est élué en présence d'une forte concentration saline ; lors de ce procédé, le tampon de lavage de 100-300 mM, de préférence de 200-300 mM, et le tampon d'élution de 200-500 mM, de préférence de 300-400 mM, contient du phosphate de sodium ou de potassium.

4. Procédé conforme à l'une des revendications de 1 à 3, **caractérisé par le fait que** la chromatographie est réalisée en cas de pH compris entre 5 et 7, de préférence entre 5,5 et 6,8.

5. Procédé conforme à l'une des revendications de 1 à 4, **caractérisé par le fait qu'**une solution contenant du phosphate de sodium ou de potassium est utilisée comme tampon circulant.

6. Procédé conforme à l'une des revendications de 1 à 5, **caractérisé par le fait qu'**est tout d'abord réalisée une chromatographie de filtration sur hydroxylapatite, que la fraction de filtration est de nouveau soumise à chromatographie dans des conditions de liaison et que la protéine cible est éluée comme fraction de VWF extrêmement pure.

7. Procédé conforme à l'une des revendications de 1 à 6, **caractérisé par le fait qu'**on utilise de l'hydroxylapatite céramique.

8. Procédé conforme à la revendication 7, **caractérisé par le fait que** l'hydroxylapatite est du type I ou II.

9. Procédé conforme à l'une des revendications de 1 à 8, **caractérisé par le fait qu'**une fraction de plasma préalablement purifiée est utilisée comme produit de départ.

10. Procédé conforme à l'une des revendications de 1 à 9, **caractérisé par le fait qu'**une solution de précipité cryogénique ultérieurement purifiée est utilisée comme produit de départ.

11. Procédé conforme à l'une des revendications de 1 à 10, **caractérisé par le fait qu'**une solution cryogénique précipitée avec de l'hydroxyde d'aluminium est utilisée comme produit de départ.

12. Procédé conforme à l'une des revendications de 1 à 11, **caractérisé par le fait qu'**une solution cryogénique précipitée avec de l'hydroxyde d'aluminium préalablement purifiée par chromatographie est utilisée comme produit de départ.

13. Procédé conforme à l'une des revendications de 1 à 12, **caractérisé par le fait que**, avant la chromatographie sur hydroxylapatite, une précipitation de pH est réalisée pour obtenir la séparation de la fibronectine.

14. Procédé conforme à l'une des revendications de 1 à 8, **caractérisé par le fait qu'**une solution de protéines et de VWF recombinant est utilisée comme produit de départ.

15. Utilisation d'hydroxylapatite pour produire une préparation de VWF à forte activité spécifique d'au moins 50 E/mg de VWF antigène.
